# EUROPEAN PATENT SPECIFICATION

(11) **EP 4 220 218 B1**
(45) Date of publication and mention of the grant of the patent: **30.07.2025**
(21) Application number: 22168126.5
(22) Date of filing: 13.04.2022
(51) Int. Cl.: G01S 7/41, G01S 13/88, G01S 13/536, G01S 13/74, G01S 13/75, G01S 13/86, G01S 7/03, H01Q 9/04, G01N 27/22, A61B 5/0507

(54) **RADIO FREQUENCY RADAR DEVICE AND METHOD FOR DETECTING VITAL INFORMATION AND HUMIDITY**
FUNKFREQUENZRADARVORRICHTUNG UND VERFAHREN ZUR ERFASSUNG VON LEBENSWICHTIGEN INFORMATIONEN UND FEUCHTIGKEIT
DISPOSITIF RADAR À RADIO FRÉQUENCE ET PROCÉDÉ DE DÉTECTION D'INFORMATIONS VITALES ET D'HUMIDITÉ

(30) Priority: 28.01.2022 TW 111104101; 28.01.2022 TW 111104102
(43) Date of publication of application: 02.08.2023
(73) Proprietor: Wistron Corporation, New Taipei City 22181 (TW)
(72) Inventor: CHANG, Yao-Tsung, 22181 New Taipei City (TW); CHEN, Yin-Yu, 22181 New Taipei City (TW); KAO, Chuan-Yen, 22181 New Taipei City (TW); LIAO, Cheng-Nung, 22181 New Taipei City (TW)
(74) Representative: Viering, Jentschura & Partner mbB Patent- und Rechtsanwälte

(56) References cited:
- US-A1- 2014 163 343
- US-A1- 2020 261 278

## Description

### BACKGROUND

### Technical Field

The present invention relates to radar signal processing technologies, and in particular, to a radio frequency radar device and a method for detecting vital information and humidity.

### Related Art

At present, although there are physiological information detection radars for detecting breathing and heartbeat, in applications such as patient care, long-term care of the elderly, and infant care, the radars are still incapable of detecting the humidity of diapers for the patients, the elderly and infants who need to wear diapers.

US 2014/163343A1 describes an apparatus, a system, and a method for monitoring the motion, breathing, heart rate and sleep state of subjects, e.g., humans. More particularly, the motion, breathing, and heart rate signals are obtained through processing applied to a raw signal obtained by a radio-frequency sensor. Periods of sleep disturbed respiration, or central apnea can be detected through analysis of the respiratory signal. The mean heart rate, and derived information, such as the presence of cardiac arrhythmias can be determined from the cardiac signal. Motion estimates can be used to recognize disturbed sleep and periodic limb movements. The sleep state may be determined by applying a classifier model to the resulting streams of respiratory, cardiac and motion data.

US 2020/261278A1 describes a wireless communication device including: a circuit unit; and an antenna which is connected to the circuit unit and which transmits and receives signals to and from a transceiver in a non-contact manner. The wireless communication device transmits different signals to the transceiver, depending on the presence or absence of contact between at least a part of the circuit unit and moisture.

### SUMMARY

The invention is defined in independent method claims 1 to 3 and corresponding apparatus claims 8, 10 and 11.

According to some embodiments, a method for detecting vital information and humidity includes: transmitting two incident radar signals with different frequencies to a field, and receiving a reflected radar signal corresponding to the field; processing the reflected radar signal to obtain a body reflection signal of an object to be measured and a tag reflection signal of a humidity sensing tag; obtaining vital information according to phase information of the body reflection signal; and obtaining humidity information according to an energy intensity of the tag reflection signal, wherein the body reflection signal is obtained by receiving the incident radar signal with a first frequency and demodulating the reflected radar signal according to the first frequency, and the tag reflection signal is obtained by receiving the incident radar signal with a second frequency and demodulating the reflected radar signal according to the second frequency.

According to some embodiments, a method for detecting vital information and humidity includes: transmitting an incident radar signal to a field and receiving a reflected radar signal corresponding to the field; processing the reflected radar signal to obtain a body reflection signal of an object to be measured and a tag reflection signal of a humidity sensing tag, wherein the humidity sensing tag comprises a frequency multiplication circuit, configured to multiply the frequency of the incident radar signal to generate the reflected radar signal with twice the frequency corresponding to the incident radar signal; obtaining vital information according to phase information of the body reflection signal; and obtaining humidity information according to an energy intensity of the tag reflection signal; wherein the body reflection signal is obtained by demodulating the reflected radar signal according to the frequency corresponding to the incident radar signal, and the tag reflection signal is obtained by demodulating the reflected radar signal according to twice the frequency corresponding to the incident radar signal.

According to some embodiments, a method for detecting vital information and humidity includes: transmitting an incident radar signal to a field and receiving a reflected radar signal corresponding to the field; processing the reflected radar signal to obtain a body reflection signal of an object to be measured and a tag reflection signal of a humidity sensing tag, wherein the humidity sensing tag comprises an input antenna, an oscillator and a frequency mixer coupled between the input antenna and the oscillator, and is configured to generate the reflected radar signal with the same frequency as the incident radar signal, and an oscillation frequency of the oscillator is different than a frequency of vital sign to be measured; obtaining vital information according to phase information of the body reflection signal; and obtaining humidity information according to an energy intensity of the tag reflection signal; wherein the body reflection signal and the tag reflection signal are distinguished according to a phase oscillation frequency of the reflected radar signal.

According to some embodiments, a radio frequency radar device includes: a transmission unit, a receiving unit, a demodulation unit, and a processing unit. The transmission unit is configured to transmit an incident radar signal to a field. The receiving unit is configured to receive a reflected radar signal corresponding to the field. The demodulation unit is coupled to the transmission unit and the receiving unit, and is configured to process the reflected radar signal to obtain a body reflection signal of an object to be measured and a tag reflection signal of a humidity sensing tag. The humidity sensing tag comprises a frequency multiplication circuit, configured to multiply the frequency of the incident radar signal to generate the reflected radar signal with twice the frequency corresponding to the incident radar signal. The processing unit is coupled to the transmission unit, the receiving unit, and the demodulation unit, and is configured to obtain vital information according to phase information of the body reflection signal, and obtain humidity information according to an energy intensity of the tag reflection signal. The body reflection signal (Sb) is obtained by demodulating the reflected radar signal (FN, FN') according to the frequency corresponding to the incident radar signal (FH), and the tag reflection signal (St) is obtained by demodulating the reflected radar signal (FN, FN') according to twice the frequency corresponding to the incident radar signal (FH).

According to some embodiments, a radio frequency radar device includes: a transmission unit, a receiving unit, a demodulation unit, and a processing unit. The transmission unit is configured to transmit an incident radar signal to a field. The receiving unit is configured to receive a reflected radar signal corresponding to the field. The demodulation unit is coupled to the transmission unit and the receiving unit, and is configured to process the reflected radar signal to obtain a body reflection signal of an object to be measured and a tag reflection signal of a humidity sensing tag. The humidity sensing tag comprises an input antenna, an oscillator and a frequency mixer coupled between the input antenna and the oscillator, and is configured to generate the reflected radar signal with the same frequency as the incident radar signal, and an oscillation frequency of the oscillator is different than a frequency of vital sign to be measured. The processing unit is coupled to the transmission unit, the receiving unit, and the demodulation unit, and is configured to obtain vital information according to phase information of the body reflection signal, and obtain humidity information according to an energy intensity of the tag reflection signal. The processing unit distinguishes the body reflection signal and the tag reflection signal according to a phase oscillation frequency of the reflected radar signal.

According to some embodiments, a radio frequency radar device includes a transmission unit, a receiving unit, a first demodulation circuit, a second demodulation circuit, and a processing unit. The transmission unit is configured to transmit two incident radar signals with different frequencies to a field. The receiving unit is configured to receive a reflected radar signal corresponding to the field. The first demodulation circuit is coupled to the transmission unit and the receiving unit, receives the incident radar signal with a first frequency, and is configured to demodulate the reflected radar signal according to the first frequency, to obtain a body reflection signal. The second demodulation circuit is coupled to the transmission unit and the receiving unit, receives the incident radar signal with a second frequency, and is configured to demodulate the reflected radar signal according to the second frequency, to obtain a tag reflection signal. The processing unit is coupled to the transmission unit, the receiving unit, the first demodulation circuit, and the second demodulation circuit, and is configured to obtain vital information according to phase information of the body reflection signal, and obtain humidity information according to an energy intensity of the tag reflection signal.

To sum up, the radio frequency radar device and the method for detecting vital information and humidity according to some embodiments can measure vital information and humidity at the same time, thereby saving device space and hardware cost.

### BRIEF DESCRIPTION OF THE DRAWINGS

FIG. 1 is a schematic diagram of a use state of a radio frequency radar device according to some embodiments;
FIG. 2 is a schematic architectural diagram of the radio frequency radar device according to some embodiments;
FIG. 3 is a flowchart of a method for detecting vital information and humidity according to some embodiments;
FIG. 4 is a schematic architectural diagram of a humidity sensing tag according to a first embodiment;
FIG. 5 is a schematic structural diagram of a humidity detection antenna according to an embodiment;
FIG. 6 is a schematic structural diagram of a humidity sensor according to an embodiment;
FIG. 7 is a schematic structural diagram of a humidity sensor according to another embodiment;
FIG. 8 is a schematic architectural diagram of the radio frequency radar device according to the first embodiment;
FIG. 9 is a frequency response diagram of the humidity detection antenna shown in FIG. 5 under different humidity environments;
FIG. 10 is a schematic diagram of a signal intensity of a tag reflection signal when the humidity detection antenna shown in FIG. 5 is under different humidity environments;
FIG. 11 is a schematic architectural diagram of the radio frequency radar device according to a second embodiment;
FIG. 12 is a schematic architectural diagram of a humidity sensing tag according to the second embodiment;
FIG. 13 is a flowchart of a method for detecting vital information and humidity according to a third embodiment; and
FIG. 14 is a schematic diagram of time-division multi-tasking according to some embodiments.

### DETAILED DESCRIPTION

The term "couple" used in this specification means that two or more components are in direct physical or electrical contact with each other, or are in indirect physical or electrical contact with each other.

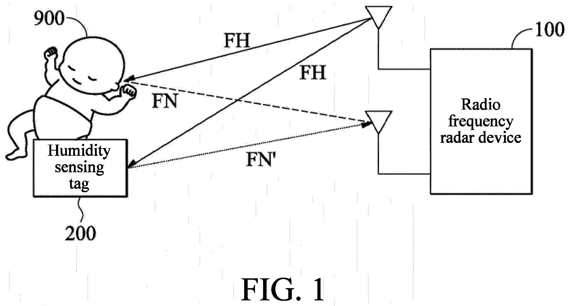

Referring to FIG. 1, FIG. 1 is a schematic diagram of a use state of a radio frequency radar device 100 according to some embodiments. The radio frequency radar device 100 transmits a radar signal (hereinafter referred to as "incident radar signal FH"). The incident radar signal FH is transmitted to a field, and is reflected back to the radio frequency radar device 100 through an object 900 to be measured, a humidity sensing tag 200, the environment, and the like. Hereinafter, the radar signal reflected by the object 900 to be measured is referred to as "reflected radar signal FN", and the radar signal reflected by the humidity sensing tag 200 is referred to as "reflected radar signal FN‴.

In some embodiments, the radio frequency radar device 100 is a continuous wave (CW) radar.

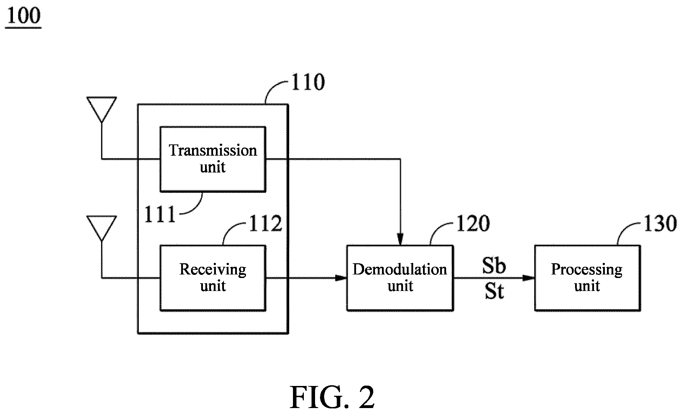

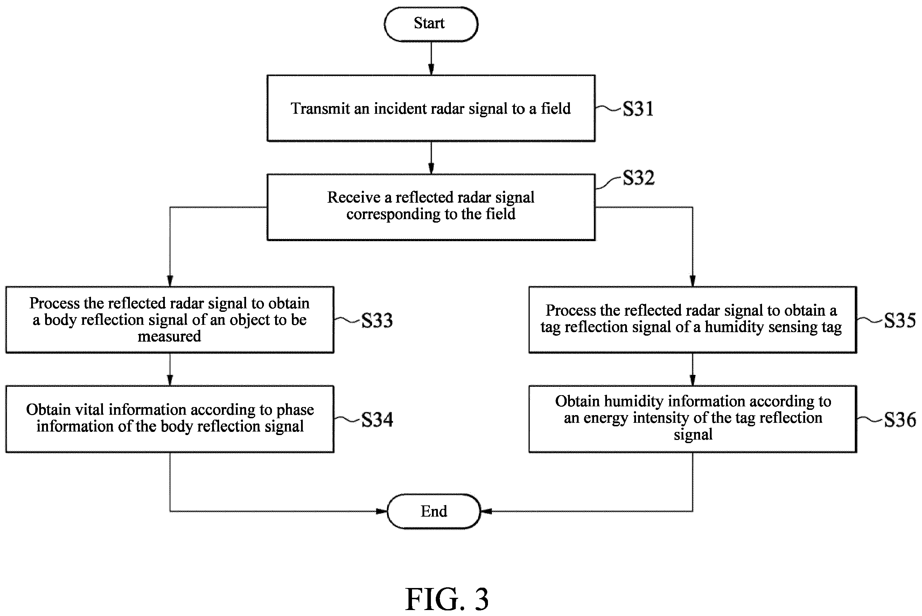

Referring to FIG. 2 and FIG. 3 together, FIG. 2 is a schematic architectural diagram of the radio frequency radar device 100 according to some embodiments, and FIG. 3 is a flowchart of a method for detecting vital information and humidity according to some embodiments. The radio frequency radar device 100 includes a transceiver 110, a demodulation unit 120, and a processing unit 130. The transceiver 110 includes a transmission unit 111 and a receiving unit 112. The transmission unit 111 is configured to transmit an incident radar signal FH (step S31). The transmission unit 111 includes a transmission antenna and an oscillation circuit (not shown). The oscillation circuit may generate a radio frequency signal, and the radio frequency signal is transmitted through the transmission antenna. Therefore, the transmission antenna is designed to be capable of effectively working within a transmission frequency range. Here, the transmission antenna may be a patch antenna, but the disclosure is not limited thereto.

The receiving unit 112 is configured to receive the reflected radar signals FN and FN' (step S32). The receiving unit 112 includes a receiving antenna, and a working frequency range of the receiving antenna needs to cover the frequencies of the reflected radar signals FN and FN'. Optionally, when the working frequency range of a receiving antenna can cover the frequencies of the reflected radar signals FN and FN', one receiving antenna is available. Conversely, two receiving antennas may be used, and the two receiving antennas separately work in different frequency ranges, so as to cover the frequencies of the reflected radar signals FN and FN' respectively. It can be understood that if only the reflected radar signals FN and FN' with the same frequency are received, there may alternatively be only one receiving antenna.

The demodulation unit 120 is coupled to the transmission unit 111 and the receiving unit 112, and is configured to process the reflected radar signals FN and FN' to obtain a body reflection signal Sb of the object 900 to be measured (step S33) and obtain a tag reflection signal St of the humidity sensing tag 200 (step S35). The processing unit 130 is coupled to the transmission unit 111, the receiving unit 112, and the demodulation unit 120 to control these units, and is configured to obtain vital information according to phase information of the body reflection signal Sb (step S34), and obtain humidity information according to an energy intensity of the tag reflection signal St (step S36). In this way, the vital information and humidity information may be acquired at the same time by the radio frequency radar device 100. For example, if the disclosure is applied to baby care, a vital response and a diaper condition of a baby may be obtained at the same time. However, the invention is not limited to this application.

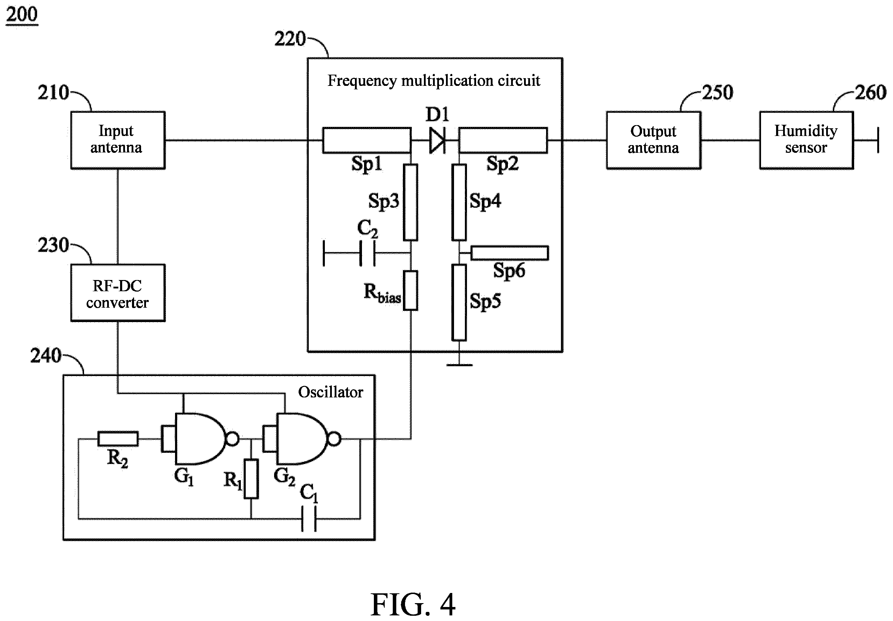

Before an operation principle of the demodulation unit 120 and the processing unit 130 is described in detail, an architecture of the humidity sensing tag 200 is described first. Referring to FIG. 4, FIG. 4 is a schematic architectural diagram of the humidity sensing tag 200 according to a first embodiment. The humidity sensing tag 200 includes an input antenna 210, a frequency multiplication circuit 220, an RF-DC converter 230, an oscillator 240, an output antenna 250, and a humidity sensor 260.

The input antenna 210 is used for receiving the incident radar signal FH delivered from the radio frequency radar device 100. Here, the input antenna 210 may be a dual-dipole patch antenna, but the disclosure is not limited thereto. The input antenna 210 is coupled to the frequency multiplication circuit 220 and the RF-DC converter 230. The frequency multiplication circuit 220 receives the incident radar signal FH received by the input antenna 210, and multiplies the frequency of the incident radar signal FH, that is, generates a signal with a frequency of 2f0 (alternatively, referred to as frequency-multiplied signal) according to the incident radar signal FH with a frequency of f0 (alternatively, referred to as fundamental frequency signal). The RF-DC converter 230 is an energy collector, and collects the energy of the incident radar signal FH received by the input antenna 210. A part of the radio frequency signal energy is collected by the RF-DC converter 230 and boosted to a working voltage of other circuits, to provide a working voltage of the humidity sensing tag 200. Therefore, the humidity sensing tag 200 does not need to be additionally equipped with a battery. However, in some embodiments, the RF-DC converter 230 may be replaced by a battery to provide a working voltage of a circuit. The RF-DC converter 230 may be mainly implemented by a voltage multiplier composed of a plurality of diodes and a plurality of capacitors. The RF-DC converter 230 is also coupled to the oscillator 240 to supply a working voltage to the oscillator 240. The oscillator 240 is coupled to a bias end of the frequency multiplication circuit 220 to generate a specific frequency signal, so as to modulate a signal of the frequency multiplication circuit 220. The specific frequency signal may be identified as a tag. FIG. 4 exemplifies a composition of the oscillator 240, which may be implemented by components such as inverters G1 and G2, resistors R1 and R2, and a capacitor C1, but the disclosure is not limited thereto.

FIG. 4 further exemplifies a composition of the frequency multiplication circuit 220, but the disclosure is not limited thereto. The frequency multiplication circuit 220 is composed of a diode D1, a plurality of microstrip lines Sp1 to Sp6, a resistor R_{bias}, and a capacitor C2. An input end of the diode D1 is coupled to components such as the microstrip lines Sp1 and Sp3, the resistor R_{bias}, and the capacitor C2. The capacitor C2 is used as a bypass capacitor to filter out the frequency-multiplied signal (2f0). The output of the frequency multiplication circuit 220 may be started up or stopped by regulating a bias voltage of the diode D1. The resistor R_{bias} is used as a bias resistor, and is coupled to the oscillator 240 to control and modulate the frequency multiplication circuit 220. An output end of the diode D1 is coupled to the microstrip lines Sp2, Sp4, Sp5, and Sp6, and forms a high-pass filter to filter out the fundamental frequency signal (f0). Here, the length of the microstrip line Sp3 is a quarter wavelength of the fundamental frequency signal, and the length of the microstrip lines Sp4, Sp5, and Sp6 is a quarter wavelength of the frequency-multiplied signal.

The output antenna 250 is coupled to the frequency multiplication circuit 220 to transmit the radio frequency signal that has been modulated and frequency-multiplied, that is, the frequency-multiplied signal (2f0) coupled with the specific frequency signal identified as the tag is output. The output antenna 250 is coupled to the humidity sensor 260 to form a humidity detection antenna. The humidity sensor 260 is a variable capacitor. The change of the medium caused by humidity may affect the dielectric coefficient of the capacitor, which may further affect the resonance frequency of the output antenna 250, causing the energy intensity of the frequency-multiplied signal (2f0) to change. Therefore, the radio frequency radar device 100 may know the humidity change by detecting the change of the signal intensity of the frequency-multiplied signal (2f0). The humidity sensor 260 may be an interdigital capacitor or a parallel plate capacitor.

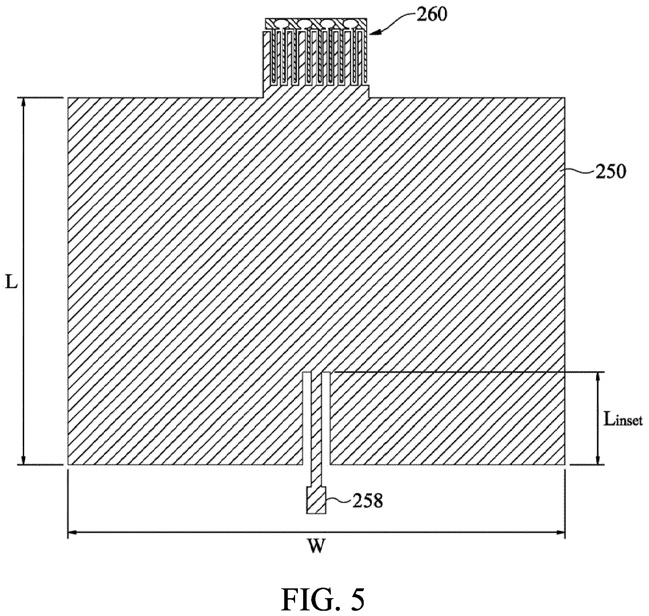

Referring to FIG. 5, FIG. 5 is a schematic structural diagram of a humidity detection antenna according to an embodiment. An endpoint 258 is a signal feed-in end of the output antenna 250, and receives the signal of the frequency multiplication circuit 220. Here, the output antenna 250 is a patch antenna with a length L of 37.5 cm, a width W of 51 cm, and a feed-in section length Lᵢₙₛₑₜ of 9.5 cm. The humidity sensor 260 is an interdigital capacitor with 18 interdigitated interdigital electrodes. Each electrode has a length of 5 cm and a width of 0.6 cm, and the electrodes are arranged at intervals of 0.3 cm. The above specifications are merely examples, and the disclosure is not limited thereto.

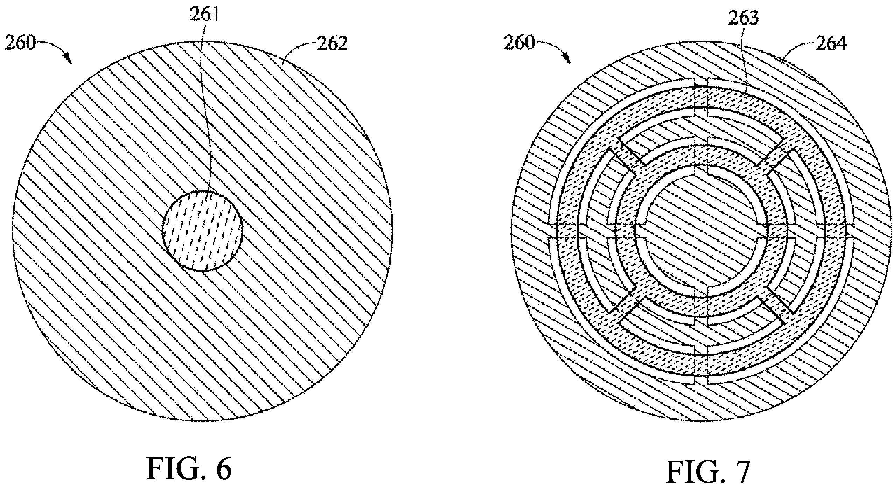

Referring to FIG. 6, FIG. 6 is a schematic structural diagram of the humidity sensor 260 according to an embodiment. Here, the humidity sensor 260 is a parallel plate capacitor having two parallel plates 261 and 262 spaced up and down.

Referring to FIG. 7, FIG. 7 is a schematic structural diagram of the humidity sensor 260 according to another embodiment. Here, the humidity sensor 260 is a ring-shaped interdigital capacitor, and includes a ring-shaped upper ring 263 and lower ring 264 disposed at an interval in a staggered manner.

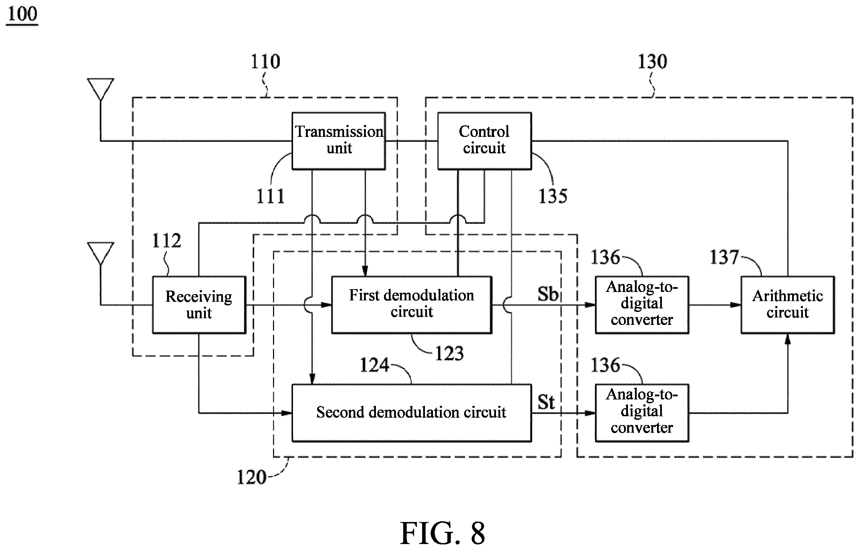

Referring to FIG. 8, FIG. 8 is a schematic architectural diagram of the radio frequency radar device 100 according to the first embodiment. Here, it should be noted that the demodulation unit 120 includes a first demodulation circuit 123 and a second demodulation circuit 124. The first demodulation circuit 123 is configured to demodulate the reflected radar signal FN reflected by the object 900 to be measured, and thus the frequency of the reflected radar signal FN is the same as that of the incident radar signal FH, which are f0. The second demodulation circuit 124 is configured to demodulate the reflected radar signal FN' backscattered by the humidity sensing tag 200. As described above, the frequency of the reflected radar signal FN' is 2f0. Because the first demodulation circuit 123 and the second demodulation circuit 124 are separately responsible for demodulating signals with different frequencies, the mutual influence between the body reflection signal Sb and the tag reflection signal St may be avoided.

The first demodulation circuit 123 is coupled to the transmission unit 111 and the receiving unit 112 to receive the incident radar signal FH from the transmission unit 111 and demodulate the reflected radar signal FN with the same frequency of f0 according to the frequency (f0) corresponding to the incident radar signal FH, to obtain the body reflection signal Sb. The body reflection signal Sb may reflect the phase information of the phase change caused by body movements of breathing and heartbeat.

The second demodulation circuit 124 is coupled to the transmission unit 111 and the receiving unit 112 to receive the frequency-multiplied incident radar signal FH (2f0) from the transmission unit 111, and is configured to demodulate the reflected radar signal FN' according to twice the frequency (2f0) corresponding to the incident radar signal FH, to obtain the tag reflection signal St. Here, the transmission unit 111 includes a frequency multiplier to generate the frequency-multiplied signal 2f0.

The processing unit 130 includes a control circuit 135, two analog-to-digital converters 136, and an arithmetic circuit 137. The control circuit 135 is coupled to the transmission unit 111, the receiving unit 112, the first demodulation circuit 123, the second demodulation circuit 124, and the arithmetic circuit 137, and is configured to control operation of these components. The two analog-to-digital converters 136 are respectively coupled to the first demodulation circuit 123 and the second demodulation circuit 124 to convert the body reflection signal Sb and the tag reflection signal St into digital signals. The arithmetic circuit 137 is coupled to the two analog-to-digital converters 136 to perform digital signal processing on the digital signals, such as removing noise, removing high-frequency signals, and removing inappropriate breathing harmonics, so as to calculate breathing and heartbeat information from the body reflection signal Sb. Specifically, vital information such as breathing and heartbeat may be obtained according to the oscillation frequency of the phase information of the body reflection signal Sb, and the respective information types may be distinguished according to their common frequency ranges. On the other hand, the arithmetic circuit 137 may determine the energy intensity of the tag reflection signal St, to identify the humidity information of the humidity sensor 260.

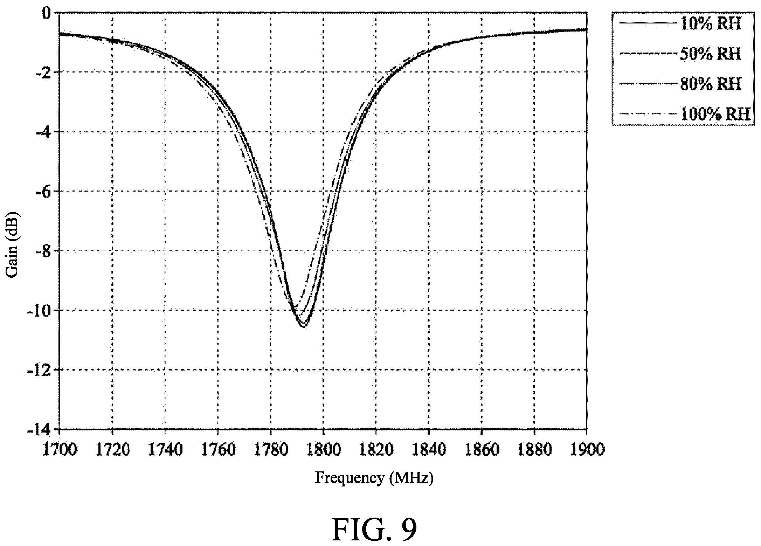

Referring to FIG. 9, FIG. 9 is a frequency response diagram of the humidity detection antenna shown in FIG. 5 under different humidity environments. A suitable resonance frequency in a dry environment is designed to be a working frequency of the tag. It can be seen that in a dry environment, the antenna circuit of the humidity detection antenna is well coordinated, and thus the intensity of the reflected radar signal FN' is high. In a humid environment, the coordination of the antenna circuit of the humidity detection antenna becomes poor, and thus the intensity of the reflected radar signal FN' becomes low. Therefore, the energy intensity of the reflected radar signal FN' may be detected to determine the humidity. In addition, when the humidity reaches a certain level, the reflected radar signal FN' becomes excessively weak, so that the radio frequency radar device 100 cannot read the specific frequency used as tag identification, which can also be used as a determination criterion of humidity.

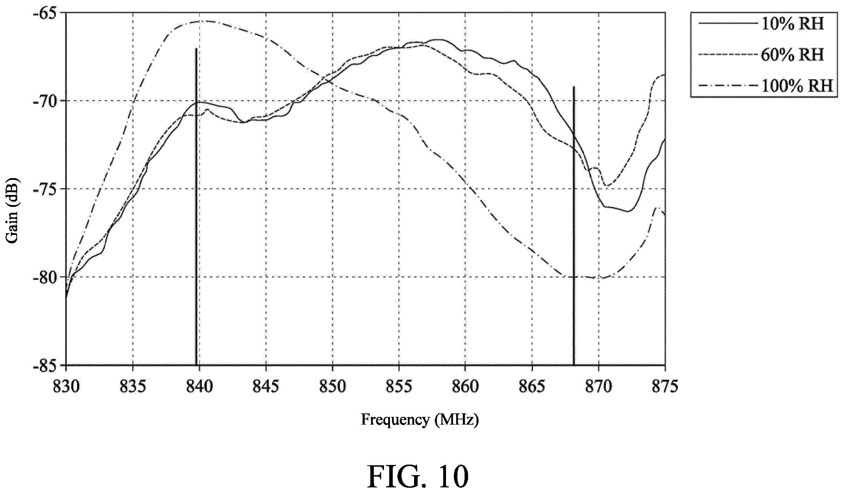

Referring to FIG. 10, FIG. 10 is a schematic diagram of a signal intensity of the tag reflection signal St when the humidity detection antenna shown in FIG. 5 is under different humidity environments. It can be seen that at different resonance frequencies, the signal intensity may gradually increase or decrease as the humidity increases. For example, the signal intensity gradually decreases at 868 MHz, and gradually increases at 840 MHz. Therefore, the humidity information may be determined according to a result of comparing the energy intensity of the tag reflection signal St with a threshold, for example, higher than or lower than a certain threshold. The threshold may also be determined in advance by experimentally measuring the change of the working frequency to be used under different humidity conditions.

In some embodiments, because the radio frequency radar device 100 and the humidity sensing tag 200 also change the energy intensity, the humidity determination may be affected. Therefore, the energy intensity in the dry environment may be recorded first when the diaper is still dry at the beginning, and then the humidity information may be determined according to the result of comparing a variation amount of the energy intensity with a threshold. Alternatively, when the radio frequency radar device 100 initially works, the current energy intensity of the reflected radar signal FN' may be recorded first, and when the variation amount of the energy intensity exceeds the threshold, it can be determined that the diaper is wet.

In some embodiments, the frequency f0 in the aforementioned first embodiment is 865 to 868 MHz, but the disclosure is not limited thereto.

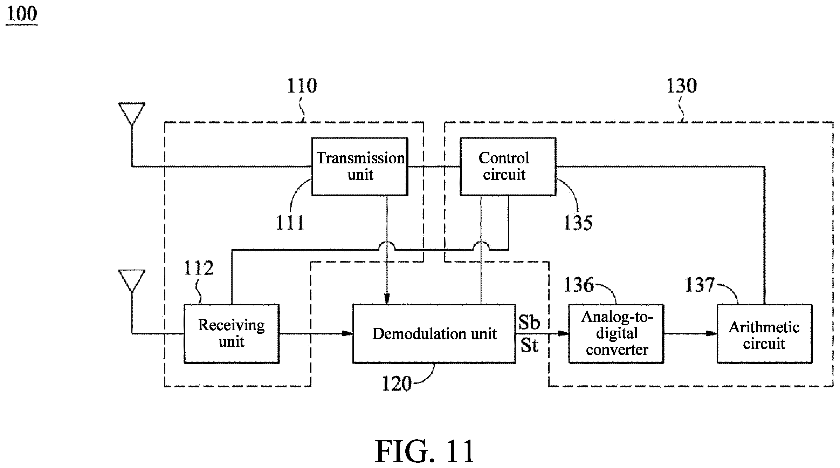

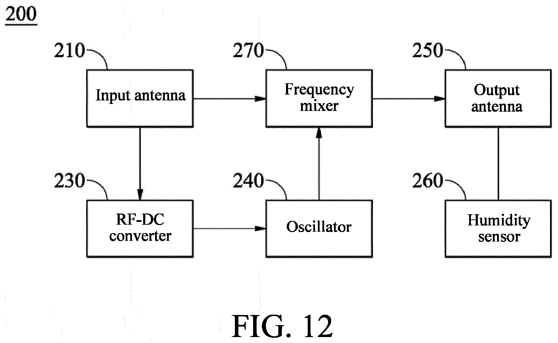

Refer to FIG. 11 and FIG. 12 together. FIG. 11 is a schematic architectural diagram of the radio frequency radar device 100 according to a second embodiment. FIG. 12 is a schematic architectural diagram of a humidity sensing tag 200 according to the second embodiment. The difference from the aforementioned first embodiment is that the humidity sensing tag 200 of this embodiment does not have the frequency multiplication circuit 220, but has a frequency mixer 270 instead, and thus the frequency of the reflected radar signal FN' is the same as that of the reflected radar signal FN and the incident radar signal FH, and all of them have a frequency f0. Therefore, the demodulation unit 120 of this embodiment is not divided into the first demodulation circuit 123 and the second demodulation circuit 124, but is only a demodulation circuit for demodulating the signal according to the frequency (f0) of the incident radar signal FH to obtain the body reflection signal Sb and the tag reflection signal St. Therefore, the hardware cost may be reduced. In order to avoid mutual influence between the reflected radar signals FN and FN', the oscillation frequency of the oscillator 240 should avoid the frequencies of breathing and heartbeat. Therefore, the body reflection signal Sb and the tag reflection signal St may be distinguished according to phase oscillation frequencies of the reflected radar signals FN and FN' (that is, the change frequency of the phase information).

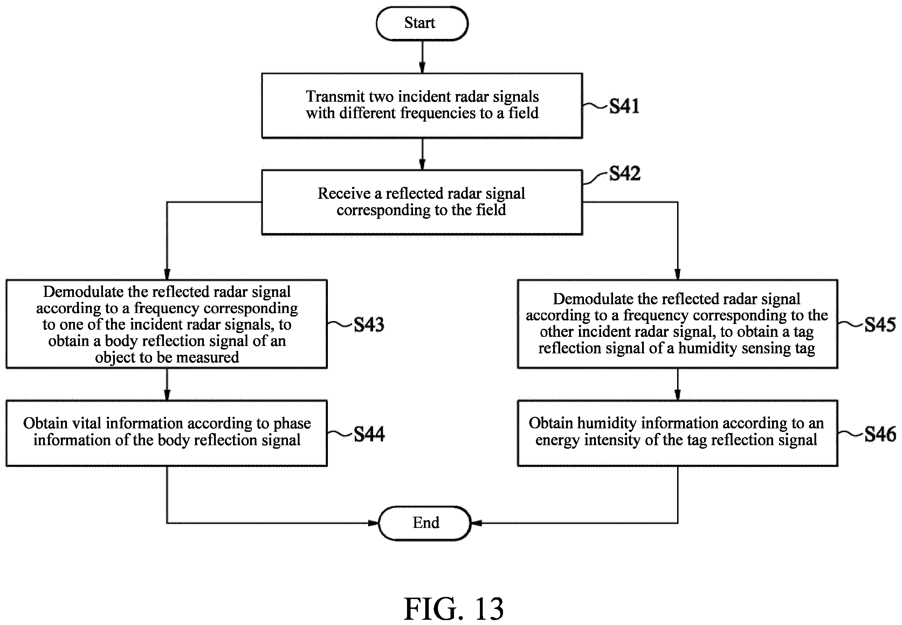

FIG. 13 is a flowchart of a method for detecting vital information and humidity according to a third embodiment. The radio frequency radar device 100 of the aforementioned first embodiment receives two frequency signals, namely f0 and 2f0 respectively, and the radio frequency radar device 100 of the second embodiment receives a frequency signal f0. Similarly, the radio frequency radar device 100 of the third embodiment receives signals with two different frequencies, but one frequency is not twice the other frequency. In some embodiments, a frequency band used by radars commonly used for measuring vital information, such as 2.4 GHz or 5 GHz, may be used as the frequency of the incident radar signal FH for measuring the body reflection signal Sb. Therefore, the detection sensitivity may be improved. On the other hand, the frequency band of 865 to 868 MHz suitable for the tag is used as the frequency of the incident radar signal FH for measuring the tag reflection signal St. Here, an antenna of the transmission unit 111 includes a millimeter-wave antenna (such as an Antipodal Vivaldi antenna) and an ultra-high frequency (UHF) band antenna.

Referring to FIG. 13, in step S41, two incident radar signals FH with different frequencies are transmitted to the field. Next, in step S42, the reflected radar signals FN and FN' corresponding to the field are received. Subsequently, the reflected radar signal FN is demodulated according to a frequency of one of the incident radar signals FH to obtain the body reflection signal Sb (step S43), and the vital information is obtained according to the phase information of the body reflection signal Sb (step S44). On the other hand, the reflected radar signal FN' is demodulated according to the frequency of the other incident radar signal FH to obtain the tag reflection signal St (step S45), and the humidity information is obtained according to the energy intensity of the tag reflection signal St (step S46).

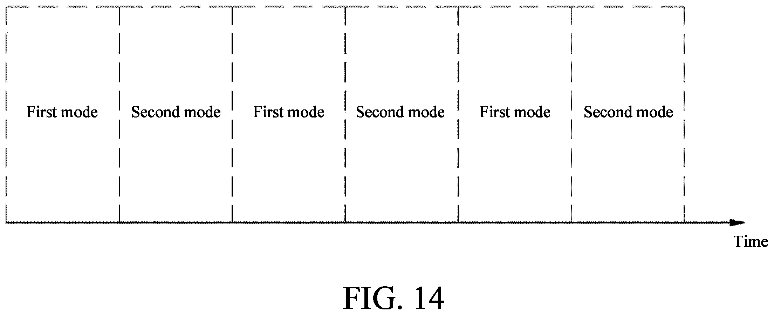

Referring to FIG.14, FIG. 14 is a schematic diagram of time-division multi-tasking according to some embodiments. The two incident radar signals FH in the third embodiment may be transmitted in a time-division duplex manner. In a first mode, the incident radar signal FH for measuring the body reflection signal Sb is transmitted, and in a second mode, the incident radar signal FH for measuring the tag reflection signal St is transmitted.

In some embodiments, the control circuit 135, the two analog-to-digital converters 136, and the arithmetic circuit 137 may be independent or be integrated together, and may be components such as a processor, a microprocessor, and a system-on-chip.

To sum up, the radio frequency radar device and the method for detecting vital information and humidity according to some embodiments can measure vital information and humidity at the same time, thereby saving device space and hardware cost.

## Claims

1. A method for detecting vital information and humidity, wherein the method for detecting vital information and humidity comprises:
transmitting two incident radar signals (FH) with different frequencies to a field and receiving a reflected radar signal (FN, FN') corresponding to the field;
processing the reflected radar signal (FN, FN') to obtain a body reflection signal (Sb) of an object (900) to be measured and a tag reflection signal (St) of a humidity sensing tag (200);
wherein the body reflection signal is obtained by receiving the incident radar signal with a first frequency and demodulating the reflected radar signal according to the first frequency, and the tag reflection signal is obtained by receiving the incident radar signal with a second frequency and demodulating the reflected radar signal according to the second frequency,
obtaining vital information according to phase information of the body reflection signal (Sb); and
obtaining humidity information according to an energy intensity of the tag reflection signal (St).

2. A method for detecting vital information and humidity, wherein the method for detecting vital information and humidity comprises:
transmitting an incident radar signal (FH) to a field and receiving a reflected radar signal (FN, FN') corresponding to the field;
processing the reflected radar signal (FN, FN') to obtain a body reflection signal (Sb) of an object (900) to be measured and a tag reflection signal (St) of a humidity sensing tag (200), wherein the humidity sensing tag (200) comprises a frequency multiplication circuit (220), configured to multiply the frequency of the incident radar signal (FH) to generate the reflected radar signal (FN') with twice the frequency corresponding to the incident radar signal (FH);
obtaining vital information according to phase information of the body reflection signal (Sb); and
obtaining humidity information according to an energy intensity of the tag reflection signal (St);
wherein the body reflection signal (Sb) is obtained by demodulating the reflected radar signal (FN, FN') according to the frequency corresponding to the incident radar signal (FH), and the tag reflection signal (St) is obtained by demodulating the reflected radar signal (FN, FN') according to twice the frequency corresponding to the incident radar signal (FH).

3. A method for detecting vital information and humidity, wherein the method for detecting vital information and humidity comprises:
transmitting an incident radar signal (FH) to a field and receiving a reflected radar signal (FN, FN') corresponding to the field;
processing the reflected radar signal (FN, FN') to obtain a body reflection signal (Sb) of an object (900) to be measured and a tag reflection signal (St) of a humidity sensing tag (200), wherein the humidity sensing tag (200) comprises an input antenna (210), an oscillator (240) and a frequency mixer (270) coupled between the input antenna (210) and the oscillator (240), and is configured to generate the reflected radar signal (FN') with the same frequency as the incident radar signal (FH), and an oscillation frequency of the oscillator (240) is different than a frequency of vital sign to be measured;
obtaining vital information according to phase information of the body reflection signal (Sb); and
obtaining humidity information according to an energy intensity of the tag reflection signal (St);
wherein the body reflection signal (Sb) and the tag reflection signal (St) are distinguished according to a phase oscillation frequency of the reflected radar signal (FN, FN').

4. The method for detecting vital information and humidity according to claim 3, wherein the body reflection signal (Sb) and the tag reflection signal (St) are obtained by demodulating the reflected radar signal (FN, FN') according to a frequency corresponding to the incident radar signal (FH).

5. The method for detecting vital information and humidity according to any of claim 1-4, wherein the humidity information is determined according to a result of comparing the energy intensity of the tag reflection signal (St) with a threshold.

6. The method for detecting vital information and humidity according to any of claim 1-4, wherein the humidity information is determined according to a result of comparing a variation amount of the energy intensity of the tag reflection signal (St)with a threshold. (FH).

7. The method for detecting vital information and humidity according to claim 1, wherein the two incident radar signals (FH) are transmitted in a time-division duplex manner.

8. A radio frequency radar device (100), wherein the radio frequency radar device (100) comprises:
a transmission unit (111), configured to transmit an incident radar signal (FH) to a field;
a receiving unit (112), configured to receive a reflected radar signal (FN, FN') corresponding to the field;
a demodulation unit (120), coupled to the transmission unit (111) and the receiving unit (112), and configured to process the reflected radar signal (FN, FN') to obtain a body reflection signal (Sb) of an object (900) to be measured and a tag reflection signal (St) of a humidity sensing tag (200), wherein the humidity sensing tag (200) comprises a frequency multiplication circuit (220), configured to multiply the frequency of the incident radar signal (FH) to generate the reflected radar signal (FN') with twice the frequency corresponding to the incident radar signal (FH); and
a processing unit (130), coupled to the transmission unit (111), the receiving unit (112), and the demodulation unit (120), and configured to obtain vital information according to phase information of the body reflection signal (Sb), and obtain humidity information according to an energy intensity of the tag reflection signal (St);
wherein the body reflection signal (Sb) is obtained by demodulating the reflected radar signal (FN, FN') according to the frequency corresponding to the incident radar signal (FH), and the tag reflection signal (St) is obtained by demodulating the reflected radar signal (FN, FN') according to twice the frequency corresponding to the incident radar signal (FH).

9. The radio frequency radar device (100) according to claim 8, wherein the demodulation unit (120) comprises:
a first demodulation circuit (123), coupled to the transmission unit (111) and the receiving unit (112), receiving the incident radar signal (FH) from the transmission unit (111), and configured to demodulate the reflected radar signal (FN, FN') according to a frequency corresponding to the incident radar signal (FH), to obtain the body reflection signal (Sb); and
a second demodulation circuit (124), coupled to the transmission unit (111) and the receiving unit (112), receiving the frequency-multiplied incident radar signal (FH) from the transmission unit (111), and configured to demodulate the reflected radar signal (FN, FN') according to twice the frequency corresponding to the incident radar signal (FH), to obtain the tag reflection signal (St).

10. A radio frequency radar device (100), wherein the radio frequency radar device (100) comprises:
a transmission unit (111), configured to transmit an incident radar signal (FH) to a field;
a receiving unit (112), configured to receive a reflected radar signal (FN, FN') corresponding to the field;
a demodulation unit (120), coupled to the transmission unit (111) and the receiving unit (112), and configured to process the reflected radar signal (FN, FN') to obtain a body reflection signal (Sb) of an object (900) to be measured and a tag reflection signal (St) of a humidity sensing tag (200), wherein the humidity sensing tag (200) comprises an input antenna (210), an oscillator (240) and a frequency mixer (270) coupled between the input antenna (210) and the oscillator (240), and is configured to generate the reflected radar signal (FN') with the same frequency as the incident radar signal (FH), and an oscillation frequency of the oscillator(240) is different than a frequency of vital sign to be measured; and
a processing unit (130), coupled to the transmission unit (111), the receiving unit (112), and the demodulation unit (120), and configured to obtain vital information according to phase information of the body reflection signal (Sb), and obtain humidity information according to an energy intensity of the tag reflection signal (St);
wherein the processing unit (130) distinguishes the body reflection signal (Sb) and the tag reflection signal (St) according to a phase oscillation frequency of the reflected radar signal (FN, FN').

11. A radio frequency radar device (100), wherein the radio frequency radar device (100) comprises:
a transmission unit (111), configured to transmit two incident radar signals (FH) with different frequencies to a field;
a receiving unit (112), configured to receive a reflected radar signal (FN, FN') corresponding to the field;
a first demodulation circuit (123), coupled to the transmission unit (111) and the receiving unit (112), receiving the incident radar signal (FH) with a first frequency, and configured to demodulate the reflected radar signal (FN, FN') according to the first frequency, to obtain a body reflection signal (Sb);
a second demodulation circuit (124), coupled to the transmission unit (111) and the receiving unit (112), receiving the incident radar signal (FH) with a second frequency, and configured to demodulate the reflected radar signal (FN, FN') according to the second frequency, to obtain a tag reflection signal (St); and
a processing unit (130), coupled to the transmission unit (111), the receiving unit (112), the first demodulation circuit (123), and the second demodulation circuit (124), and configured to obtain vital information according to phase information of the body reflection signal (Sb), and obtain humidity information according to an energy intensity of the tag reflection signal (St).

12. The radio frequency radar device (100) according to claim 11, wherein the processing unit (130) determines the humidity information according to a result of comparing the energy intensity of the tag reflection signal (St) with a threshold.

13. The radio frequency radar device (100) according to claim 11, wherein the processing unit (130) determines the humidity information according to a result of comparing a variation amount of the energy intensity of the tag reflection signal (St) with a threshold.

## Patentansprüche

1. Verfahren zum Detektieren von Vitalinformationen und Feuchtigkeit, wobei das Verfahren zum Detektieren von Vitalinformationen und Feuchtigkeit aufweist:
Übertragen von zwei einfallenden Funksignalen (FH) mit unterschiedlichen Frequenzen hin zu einem Feld und Empfangen eines reflektierten Funksignals (FN, FN'), welches mit dem Feld korrespondiert;
Verarbeiten des reflektierten Funksignals (FN, FN'), um ein Körperreflexionssignal (Sb) eines zu messenden Objekts (900) und ein Etikett-Reflexionssignal (St) eines Feuchtigkeitsmessung-Etiketts (200) zu erlangen;
wobei das Körperreflexionssignal erlangt wird mittels Empfangens des einfallenden Funksignals mit einer ersten Frequenz und Demodulierens des reflektierten Funksignals gemäß der ersten Frequenz, und wobei das Etikett-Reflexionssignal erlangt wird mittels Empfangens des einfallenden Funksignals mit einer zweiten Frequenz und Demodulierens des reflektierten Funksignals gemäß der zweiten Frequenz,
Erlangen von Vitalinformationen gemäß Phaseninformationen des Körperreflexionssignals (Sb); und
Erlangen von Feuchtigkeitsinformationen gemäß einer Energie-Intensität des Etikett-Reflexionssignals (St).

2. Verfahren zum Detektieren von Vitalinformationen und Feuchtigkeit, wobei das Verfahren zum Detektieren von Vitalinformationen und Feuchtigkeit aufweist:
Übertragen eines einfallenden Funksignals (FH) hin zu einem Feld und Empfangen eines reflektierten Funksignals (FN, FN'), welches mit dem Feld korrespondiert;
Verarbeiten des reflektierten Funksignals (FN, FN'), um ein Körperreflexionssignal (Sb) eines zu messenden Objekts (900) und ein Etikett-Reflexionssignal (St) eines Feuchtigkeitsmessung-Etiketts (200) zu erlangen, wobei das Feuchtigkeitsmessung-Etikett (200) einen Frequenzmultiplikationsschaltkreis (220) aufweist, welcher konfiguriert ist, um die Frequenz des einfallenden Funksignals (FH) zu multiplizieren, um das reflektierte Funksignal (FN') mit der doppelten Frequenz zu erzeugen, welche mit dem einfallenden Funksignal (FH) korrespondiert;
Erlangen von Vitalinformationen gemäß Phaseninformationen des Körperreflexionssignals (Sb); und
Erlangen von Feuchtigkeitsinformationen gemäß einer Energie-Intensität des Etikett-Reflexionssignals (St);
wobei das Körperreflexionssignal (Sb) erlangt wird mittels Demodulierens des reflektierten Funksignals (FN, FN') gemäß der Frequenz, welche mit dem einfallenden Funksignal (FH) korrespondiert, und wobei das Etikett-Reflexionssignal (St) erlangt wird mittels Demodulierens des reflektierten Funksignals (FN, FN') gemäß der doppelten Frequenz, welche mit dem einfallenden Funksignal (FH) korrespondiert.

3. Verfahren zum Detektieren von Vitalinformationen und Feuchtigkeit, wobei das Verfahren zum Detektieren von Vitalinformationen und Feuchtigkeit aufweist:
Übertragen eines einfallenden Funksignals (FH) hin zu einem Feld und Empfangen eines reflektierten Funksignals (FN, FN'), welches mit dem Feld korrespondiert;
Verarbeiten des reflektierten Funksignals (FN, FN'), um ein Körperreflexionssignal (Sb) eines zu messenden Objekts (900) und ein Etikett-Reflexionssignal (St) eines Feuchtigkeitsmessung-Etiketts (200) zu erlangen, wobei das Feuchtigkeitsmessung-Etikett (200) eine Eingangsantenne (210), eine Oszillationsvorrichtung (240) und eine Frequenzmischungsvorrichtung (270) aufweist, welche zwischen der Eingangsantenne (210) und der Oszillationsvorrichtung (240) gekoppelt ist und konfiguriert ist, um das reflektierte Funksignal (FN') mit der gleichen Frequenz wie das einfallende Funksignal (FH) zu erzeugen, und wobei sich eine Oszillationsfrequenz der Oszillationsvorrichtung (240) von einer Frequenz eines zu messenden Vitalzeichens unterscheidet;
Erlangen von Vitalinformationen gemäß Phaseninformationen des Körperreflexionssignals (Sb); und
Erlangen von Feuchtigkeitsinformationen gemäß einer Energie-Intensität des Etikett-Reflexionssignals (St);
wobei das Körperreflexionssignal (Sb) und das Etikett-Reflexionssignal (St) gemäß einer Phasenoszillationsfrequenz des reflektierten Funksignals (FN, FN') unterschieden werden.

4. Verfahren zum Detektieren von Vitalinformationen und Feuchtigkeit gemäß Anspruch 3, wobei das Körperreflexionssignal (Sb) und das Etikett-Reflexionssignal (St) erlangt werden mittels Demodulierens des reflektierten Funksignals (FN, FN') gemäß einer Frequenz, welche mit dem einfallenden Funksignal (FH) korrespondiert.

5. Verfahren zum Detektieren von Vitalinformationen und Feuchtigkeit gemäß einem der Ansprüche 1 bis 4, wobei die Feuchtigkeitsinformationen gemäß einem Ergebnis eines Vergleichens der Energie-Intensität des Etikett-Reflexionssignals (St) mit einem Schwellenwert ermittelt werden.

6. Verfahren zum Detektieren von Vitalinformationen und Feuchtigkeit gemäß einem der Ansprüche 1 bis 4, wobei die Feuchtigkeitsinformationen gemäß einem Ergebnis eines Vergleichens eines Änderungsbetrags der Energie-Intensität des Etikett-Reflexionssignals (St) mit einem Schwellenwert ermittelt werden.

7. Verfahren zum Detektieren von Vitalinformationen und Feuchtigkeit gemäß Anspruch 1, wobei die zwei einfallenden Funksignale (FH) in einer Zeitduplex-Weise übertragen werden.

8. Radiofrequenz-Funk-Vorrichtung (100), wobei die Radiofrequenz-Funk-Vorrichtung (100) aufweist:
eine Übertragungseinheit (111), welche konfiguriert ist, um ein einfallendes Funksignal (FH) hin zu einem Feld zu übertragen;
eine Empfangseinheit (112), welche konfiguriert ist, um ein reflektiertes Funksignal (FN, FN'), welches mit dem Feld korrespondiert, zu empfangen;
eine Demodulationseinheit (120), welche mit der Übertragungseinheit (111) und der Empfangseinheit (112) gekoppelt ist und konfiguriert ist, um das reflektierte Funksignal (FN, FN') zu verarbeiten, um ein Körperreflexionssignal (Sb) eines zu messenden Objekts (900) und ein Etikett-Reflexionssignal (St) eines Feuchtigkeitsmessung-Etiketts (200) zu erlangen, wobei das Feuchtigkeitsmessung-Etikett (200) einen Frequenzmultiplikationsschaltkreis (220) aufweist, welcher konfiguriert ist, um die Frequenz des einfallenden Funksignals (FH) zu multiplizieren, um das reflektierte Funksignal (FN') mit der doppelten Frequenz zu erzeugen, welche mit dem einfallenden Funksignal (FH) korrespondiert; und
eine Verarbeitungseinheit (130), welche mit der Übertragungseinheit (111), der Empfangseinheit (112) und der Demodulationseinheit (120) gekoppelt ist und konfiguriert ist, um Vitalinformationen gemäß Phaseninformationen des Körperreflexionssignals (Sb) zu erlangen und um Feuchtigkeitsinformationen gemäß einer Energie-Intensität des Etikett-Reflexionssignals (St) zu erlangen;
wobei das Körperreflexionssignal (Sb) erlangt wird mittels Demodulierens des reflektierten Funksignals (FN, FN') gemäß der Frequenz, welche mit dem einfallenden Funksignal (FH) korrespondiert, und wobei das Etikett-Reflexionssignal (St) erlangt wird mittels Demodulierens des reflektierten Funksignals (FN, FN') gemäß der doppelten Frequenz, welche mit dem einfallenden Funksignal (FH) korrespondiert.

9. Radiofrequenz-Funk-Vorrichtung (100) gemäß Anspruch 8, wobei die Demodulationseinheit (120) aufweist:
einen ersten Demodulationsschaltkreis (123), welcher mit der Übertragungseinheit (111) und der Empfangseinheit (112) gekoppelt ist, das einfallende Funksignal (FH) von der Übertragungseinheit (111) aus empfängt und konfiguriert ist, um das reflektierte Funksignal (FN, FN') gemäß einer Frequenz, welche mit dem einfallenden Funksignal (FH) korrespondiert, zu demodulieren, um das Körperreflexionssignal (Sb) zu erlangen; und
einen zweiten Demodulationsschaltkreis (124), welcher mit der Übertragungseinheit (111) und der Empfangseinheit (112) gekoppelt ist, das frequenzmultiplizierte einfallende Funksignal (FH) von der Übertragungseinheit (111) aus empfängt und konfiguriert ist, um das reflektierte Funksignal (FN, FN') gemäß der doppelten Frequenz, welche mit dem einfallenden Funksignal (FH) korrespondiert, zu demodulieren, um das Etikett-Reflexionssignal (St) zu erlangen.

10. Radiofrequenz-Funk-Vorrichtung (100), wobei die Radiofrequenz-Funk-Vorrichtung (100) aufweist:
eine Übertragungseinheit (111), welche konfiguriert ist, um ein einfallendes Funksignal (FH) hin zu einem Feld zu übertragen;
eine Empfangseinheit (112), welche konfiguriert ist, um ein reflektiertes Funksignal (FN, FN'), welches mit dem Feld korrespondiert, zu empfangen;
eine Demodulationseinheit (120), welche mit der Übertragungseinheit (111) und der Empfangseinheit (112) gekoppelt ist und konfiguriert ist, um das reflektierte Funksignal (FN, FN') zu verarbeiten, um ein Körperreflexionssignal (Sb) eines zu messenden Objekts (900) und ein Etikett-Reflexionssignal (St) eines Feuchtigkeitsmessung-Etiketts (200) zu erlangen, wobei das Feuchtigkeitsmessung-Etikett (200) eine Eingangsantenne (210), eine Oszillationsvorrichtung (240) und eine Frequenzmischungsvorrichtung (270), welche zwischen der Eingangsantenne (210) und der Oszillationsvorrichtung (240) gekoppelt ist, aufweist und konfiguriert ist, um das reflektierte Funksignal (FN') mit der gleichen Frequenz wie das einfallende Funksignal (FH) zu erzeugen, und wobei sich eine Oszillationsfrequenz der Oszillationsvorrichtung (240) von einer Frequenz eines zu messenden Vitalzeichens unterscheidet; und
eine Verarbeitungseinheit (130), welche mit der Übertragungseinheit (111), der Empfangseinheit (112) und der Demodulationseinheit (120) gekoppelt ist und konfiguriert ist, um Vitalinformationen gemäß Phaseninformationen des Körperreflexionssignals (Sb) zu erlangen und um Feuchtigkeitsinformationen gemäß einer Energie-Intensität des Etikett-Reflexionssignals (St) zu erlangen;
wobei die Verarbeitungseinheit (130) das Körperreflexionssignal (Sb) und das Etikett-Reflexionssignal (St) gemäß einer Phasenoszillationsfrequenz des reflektierten Funksignals (FN, FN') unterscheidet.

11. Radiofrequenz-Funk-Vorrichtung (100), wobei die Radiofrequenz-Funk-Vorrichtung (100) aufweist:
eine Übertragungseinheit (111), welche konfiguriert ist, um zwei einfallende Funksignale (FH) mit unterschiedlichen Frequenzen hin zu einem Feld zu übertragen;
eine Empfangseinheit (112), welche konfiguriert ist, um ein reflektiertes Funksignal (FN, FN'), welches mit dem Feld korrespondiert, zu empfangen;
einen ersten Demodulationsschaltkreis (123), welcher mit der Übertragungseinheit (111) und der Empfangseinheit (112) gekoppelt ist, das einfallende Funksignal (FH) mit einer ersten Frequenz empfängt und konfiguriert ist, um das reflektierte Funksignal (FN, FN') gemäß der ersten Frequenz zu demodulieren, um ein Körperreflexionssignal (Sb) zu erlangen;
einen zweiten Demodulationsschaltkreis (124), welcher mit der Übertragungseinheit (111) und der Empfangseinheit (112) gekoppelt ist, das einfallende Funksignal (FH) mit einer zweiten Frequenz empfängt und konfiguriert ist, um das reflektierte Funksignal (FN, FN') gemäß der zweiten Frequenz zu demodulieren, um ein Etikett-Reflexionssignal (St) zu erlangen; und
eine Verarbeitungseinheit (130), welche mit der Übertragungseinheit (111), der Empfangseinheit (112), dem ersten Demodulationsschaltkreis (123) und dem zweiten Demodulationsschaltkreis(124) gekoppelt ist und konfiguriert ist, um Vitalinformationen gemäß Phaseninformationen des Körperreflexionssignals (Sb) erlangt und um Feuchtigkeitsinformationen gemäß einer Energie-Intensität des Etikett-Reflexionssignals (St) zu erlangen.

12. Radiofrequenz-Funk-Vorrichtung (100) gemäß Anspruch 11, wobei die Verarbeitungseinheit (130) die Feuchtigkeitsinformationen gemäß einem Ergebnis eines Vergleichens der Energie-Intensität des Etikett-Reflexionssignals (St) mit einem Schwellenwert ermittelt.

13. Radiofrequenz-Funk-Vorrichtung (100) gemäß Anspruch 11, wobei die Verarbeitungseinheit (130) die Feuchtigkeitsinformationen gemäß einem Ergebnis eines Vergleichens eines Änderungsbetrags der Energie-Intensität des Etikett-Reflexionssignals (St) mit einem Schwellenwert ermittelt.

## Revendications

1. Procédé de détection d'informations vitales et d'humidité, dans lequel le procédé de détection d'informations vitales et d'humidité comprend :
la transmission de deux signaux radar incidents (FH) de fréquences différentes à un champ et la réception d'un signal radar réfléchi (FN, FN') correspondant au champ ;
le traitement du signal radar réfléchi (FN, FN') pour obtenir un signal de réflexion corporelle (Sb) d'un objet (900) à mesurer et un signal de réflexion d'étiquette (St) d'une étiquette de détection d'humidité (200) ;
dans lequel le signal de réflexion corporelle est obtenu en recevant le signal radar incident avec une première fréquence et en démodulant le signal radar réfléchi en fonction de la première fréquence, et le signal de réflexion d'étiquette est obtenu en recevant le signal radar incident avec une deuxième fréquence et en démodulant le signal radar réfléchi en fonction de la deuxième fréquence,
l'obtention d'informations vitales en fonction d'informations de phase du signal de réflexion corporelle (Sb) ; et
l'obtention d'informations d'humidité en fonction d'une intensité d'énergie du signal de réflexion d'étiquette (St).

2. Procédé de détection d'informations vitales et d'humidité, dans lequel le procédé de détection d'informations vitales et d'humidité comprend :
la transmission d'un signal radar incident (FH) à un champ et la réception d'un signal radar réfléchi (FN, FN') correspondant au champ ;
le traitement du signal radar réfléchi (FN, FN') pour obtenir un signal de réflexion corporelle (Sb) d'un objet (900) à mesurer et un signal de réflexion d'étiquette (St) d'une étiquette de détection d'humidité (200), dans lequel l'étiquette de détection d'humidité (200) comprend un circuit de multiplication de fréquence (220), configuré pour multiplier la fréquence du signal radar incident (FH) afin de générer le signal radar réfléchi (FN') avec un double de la fréquence correspondant au signal radar incident (FH) ;
l'obtention d'informations vitales en fonction d'informations de phase du signal de réflexion corporelle (Sb) ; et
l'obtention d'informations d'humidité en fonction d'une intensité d'énergie du signal de réflexion d'étiquette (St) ;
dans lequel le signal de réflexion corporelle (Sb) est obtenu en démodulant le signal radar réfléchi (FN, FN') en fonction de la fréquence correspondant au signal radar incident (FH), et le signal de réflexion d'étiquette (St) est obtenu en démodulant le signal radar réfléchi (FN, FN') en fonction du double de la fréquence correspondant au signal radar incident (FH).

3. Procédé de détection d'informations vitales et d'humidité, dans lequel le procédé de détection d'informations vitales et d'humidité comprend :
la transmission d'un signal radar incident (FH) à un champ et la réception d'un signal radar réfléchi (FN, FN') correspondant au champ ;
le traitement du signal radar réfléchi (FN, FN') pour obtenir un signal de réflexion corporelle (Sb) d'un objet (900) à mesurer et un signal de réflexion d'étiquette (St) d'une étiquette de détection d'humidité (200), dans lequel l'étiquette de détection d'humidité (200) comprend une antenne d'entrée (210), un oscillateur (240) et un mélangeur de fréquences (270) couplés entre l'antenne d'entrée (210) et l'oscillateur (240), et est configurée pour générer le signal radar réfléchi (FN') avec la même fréquence que le signal radar incident (FH), et une fréquence d'oscillation de l'oscillateur (240) est différente d'une fréquence de signe vital à mesurer ;
l'obtention d'informations vitales en fonction d'informations de phase du signal de réflexion corporelle (Sb) ; et
l'obtention d'informations d'humidité en fonction d'une intensité d'énergie du signal de réflexion d'étiquette (St) ;
dans lequel le signal de réflexion corporelle (Sb) et le signal de réflexion d'étiquette (St) sont distingués en fonction d'une fréquence d'oscillation de phase du signal radar réfléchi (FN, FN').

4. Procédé de détection d'informations vitales et d'humidité selon la revendication 3, dans lequel le signal de réflexion corporelle (Sb) et le signal de réflexion d'étiquette (St) sont obtenus en démodulant le signal radar réfléchi (FN, FN') en fonction d'une fréquence correspondant au signal radar incident (FH).

5. Procédé de détection d'informations vitales et d'humidité selon l'une quelconque des revendications 1 à 4, dans lequel les informations d'humidité sont déterminées en fonction d'un résultat de comparaison de l'intensité d'énergie du signal de réflexion d'étiquette (St) avec un seuil.

6. Procédé de détection d'informations vitales et d'humidité selon l'une quelconque des revendications 1 à 4, dans lequel les informations d'humidité sont déterminées en fonction d'un résultat de comparaison d'un montant de variation de l'intensité d'énergie du signal de réflexion d'étiquette (St) avec un seuil.

7. Procédé de détection d'informations vitales et d'humidité selon la revendication 1, dans lequel les deux signaux radar incidents (FH) sont transmis en duplex par répartition dans le temps.

8. Dispositif radar à radiofréquence (100), dans lequel le dispositif radar à radiofréquence (100) comprend :
une unité de transmission (111), configurée pour transmettre un signal radar incident (FH) à un champ ;
une unité de réception (112), configurée pour recevoir un signal radar réfléchi (FN, FN') correspondant au champ ;
une unité de démodulation (120), couplée à l'unité de transmission (111) et à l'unité de réception (112), et configurée pour traiter le signal radar réfléchi (FN, FN') afin d'obtenir un signal de réflexion corporelle (Sb) d'un objet (900) à mesurer et un signal de réflexion d'étiquette (St) d'une étiquette de détection d'humidité (200), où l'étiquette de détection d'humidité (200) comprend un circuit de multiplication de fréquence (220), configuré pour multiplier la fréquence du signal radar incident (FH) afin de générer le signal radar réfléchi (FN') avec un double de la fréquence correspondant au signal radar incident (FH) ; et
une unité de traitement (130), couplée à l'unité de transmission (111), à l'unité de réception (112) et à l'unité de démodulation (120), et configurée pour obtenir des informations vitales en fonction d'informations de phase du signal de réflexion corporelle (Sb), et pour obtenir des informations d'humidité en fonction d'une intensité d'énergie du signal de réflexion d'étiquette (St) ;
dans lequel le signal de réflexion corporelle (Sb) est obtenu en démodulant le signal radar réfléchi (FN, FN') en fonction de la fréquence correspondant au signal radar incident (FH), et le signal de réflexion d'étiquette (St) est obtenu en démodulant le signal radar réfléchi (FN, FN') en fonction du double de la fréquence correspondant au signal radar incident (FH).

9. Dispositif radar à radiofréquence (100) selon la revendication 8, dans lequel l'unité de démodulation (120) comprend :
un premier circuit de démodulation (123), couplé à l'unité de transmission (111) et à l'unité de réception (112), recevant le signal radar incident (FH) provenant de l'unité de transmission (111), et configuré pour démoduler le signal radar réfléchi (FN, FN') en fonction d'une fréquence correspondant au signal radar incident (FH), afin d'obtenir le signal de réflexion corporelle (Sb) ; et
un deuxième circuit de démodulation (124), couplé à l'unité de transmission (111) et à l'unité de réception (112), recevant le signal radar incident multiplié en fréquence (FH) provenant de l'unité de transmission (111), et configuré pour démoduler le signal radar réfléchi (FN, FN') en fonction du double de la fréquence correspondant au signal radar incident (FH), afin d'obtenir le signal de réflexion d'étiquette (St).

10. Dispositif radar à radiofréquence (100), dans lequel le dispositif radar à radiofréquence (100) comprend :
une unité de transmission (111), configurée pour transmettre un signal radar incident (FH) à un champ ;
une unité de réception (112), configurée pour recevoir un signal radar réfléchi (FN, FN') correspondant au champ ;
une unité de démodulation (120), couplée à l'unité de transmission (111) et à l'unité de réception (112), et configurée pour traiter le signal radar réfléchi (FN, FN') afin d'obtenir un signal de réflexion corporelle (Sb) d'un objet (900) à mesurer et un signal de réflexion d'étiquette (St) d'une étiquette de détection d'humidité (200), où l'étiquette de détection d'humidité (200) comprend une antenne d'entrée (210), un oscillateur (240) et un mélangeur de fréquences (270) couplé entre l'antenne d'entrée (210) et l'oscillateur (240), et est configurée pour générer le signal radar réfléchi (FN') avec la même fréquence que le signal radar incident (FH), et une fréquence d'oscillation de l'oscillateur (240) est différente d'une fréquence de signe vital à mesurer ; et
une unité de traitement (130), couplée à l'unité de transmission (111), à l'unité de réception (112) et à l'unité de démodulation (120), et configurée pour obtenir des informations vitales en fonction des informations de phase du signal de réflexion corporelle (Sb), et pour obtenir des informations d'humidité en fonction d'une intensité d'énergie du signal de réflexion d'étiquette (St) ;
dans lequel l'unité de traitement (130) distingue le signal de réflexion corporelle (Sb) et le signal de réflexion d'étiquette (St) en fonction d'une fréquence d'oscillation de phase du signal radar réfléchi (FN, FN').

11. Dispositif radar à radiofréquence (100), dans lequel le dispositif radar à radiofréquence (100) comprend :
une unité de transmission (111), configurée pour transmettre deux signaux radar incidents (FH) avec des fréquences différentes à un champ ;
une unité de réception (112), configurée pour recevoir un signal radar réfléchi (FN, FN') correspondant au champ ;
un premier circuit de démodulation (123), couplé à l'unité de transmission (111) et à l'unité de réception (112), recevant le signal radar incident (FH) avec une première fréquence, et configuré pour démoduler le signal radar réfléchi (FN, FN') en fonction de la première fréquence, afin d'obtenir un signal de réflexion corporelle (Sb) ;
un deuxième circuit de démodulation (124), couplé à l'unité de transmission (111) et à l'unité de réception (112), recevant le signal radar incident (FH) avec une deuxième fréquence, et configuré pour démoduler le signal radar réfléchi (FN, FN') en fonction de la deuxième fréquence, afin d'obtenir un signal de réflexion d'étiquette (St) ; et
une unité de traitement (130), couplée à l'unité de transmission (111), à l'unité de réception (112), au premier circuit de démodulation (123) et au deuxième circuit de démodulation (124), et configurée pour obtenir des informations vitales en fonction d'informations de phase du signal de réflexion corporelle (Sb), et pour obtenir des informations d'humidité en fonction d'une intensité d'énergie du signal de réflexion d'étiquette (St).

12. Dispositif radar à radiofréquence (100) selon la revendication 11, dans lequel l'unité de traitement (130) détermine les informations d'humidité en fonction d'un résultat de comparaison de l'intensité d'énergie du signal de réflexion d'étiquette (St) avec un seuil.

13. Dispositif radar à radiofréquence (100) selon la revendication 11, dans lequel l'unité de traitement (130) détermine les informations d'humidité en fonction d'un résultat de comparaison d'un montant de variation de l'intensité d'énergie du signal de réflexion d'étiquette (St) avec un seuil.
